# EUROPÄISCHE PATENTANMELDUNG

(11) **EP 1 314 433 A1**
(43) Veröffentlichungstag der Anmeldung: **28.05.2003**
(21) Anmeldenummer: 01127914.8
(22) Anmeldetag: 23.11.2001
(51) Int. Cl.: A61K 35/78

(54) **Verfahren zur Herstellung von Extrakten des Ginkgo biloba**

(71) Anmelder: Cognis Iberia, S.L., 08755 Castellbisbal, (Barcelona) (ES)
(72) Erfinder: ALAQUI ISMAILI, Smail, 08290 Cerdanyola del Vallès (ES); RULL PROUS, Santiago, 08034 Barcelona (ES); GRANOLLERAS CASTELLO, Anna, 08013 Barcelona (ES)
(74) Vertreter: Fabry, Bernd, Dr.

(57) **Zusammenfassung**

Vorgeschlagen wird ein Verfahren zur Herstellung von Extrakten des Ginkgo biloba, umfassend die Extraktion der Blätter mit organischen oder wässrig/organischen Lösemitteln, die Adsorption der in den Extrakten enthaltenen Ginkgoflavonoide, Ginkgolide und Bilobalide an Harzen und deren Desorption durch organische Lösemittel, welches sich dadurch auszeichnet, dass man als Adsorbens phenolhaltige Harze einsetzt.

## Beschreibung

### Gebiet der Erfindung

Die Erfindung befindet sich auf dem Gebiet der botanischen Extrakten und betrifft ein Verfahren zur Herstellung von Ginkgoextrakten mit verbesserter Reinheit.

### Stand der Technik

Der Ginkgobaum wurde schon von Darwin enthusiastisch als lebendes Fossil bezeichnet, weil alle seine Eigenschaften mit Langlebigkeit verbunden zu sein scheinen. Seit Jahrhunderten werden Ginkgos in China und Japan in Tempelgärten angepflanzt und gepflegt, andernfalls wäre diese Spezies gewiss längst ausgestorben. Ebenso lange werden Extrakte von Ginkgoblättern in der traditionellen Medizin Asiens eingesetzt. In China wird beispielsweise das sogenannte "Bai-guo-ye" zur Bekämpfung so unterschiedlicher Erkrankungen wie Atemwegsbeschwerden, Hörschwäche, hohen Blutdruck, Gedächtnisverlust, Angina pectoris, Hautausschlag und Magenschmerzen eingesetzt. Die westliche Medizin erkannte die Heilkraft der Ginkgoinhaltsstoffe erst in den 50er Jahre. Schwabe analysierte erstmals die Zusammensetzung und bestimmte die Aktivität der gefundenen Stoffe. Er war auch der erste, der einen 0,1 Gew.-%igen Ginkgoextrakt unter der Bezeichnung *Tebonin* in den Handel brachte. Heutige Produkte weise eine Verdünnung von 1: 50 auf und sind unter den Handelsnamen *Kaveri, Tanakan, Rökan* oder *Ginkgold* bekannt. Die aktiven Inhaltsstoffe des Ginkgo sind vor allem Flavonoidglycoside ("Ginkgoflavonoide"), die u.a. (Iso)Quercitin, Kaempferol, Kaempferol-3-rhamnoside, Isorhamnetin, Luteolin, Luteolinglykoside, Sitosterolglycoside sowie hexacyclische Terpenlactone umfassen, die als Ginkgolide bzw. Bilobalide bezeichnet werden.

Auch die Herstellung von Ginkgoextrakten ist in der Literatur vielfach beschrieben worden. Gegenstand der **EP 0360556 B1** (Indena) ist beispielsweise die Extraktion der Blätter mit einem Lösemittelgemisch aus einem Aromaten und einem niederen Alkohol. In der Druckschrift **EP 0431536 B1** (Schwabe) wird ein mehrstufiges Verfahren beschrieben, bei dem Extraktion nacheinander mit Aceton oder niederen Alkoholen, Methylethylketon, Butanol oder Pentanol sowie schließlich Alkanen oder Cycloalkanen erfolgt. Weitere Verfahren sind beispielsweise in **US 5,885,582** (Montana) oder **ES 2036951 B1** (Euromed) beschrieben.

Von Nachteil ist dabei jedoch, dass der Wertstoffgehalt nach den Verfahren des Stands der Technik nicht oberhalb von 21 Gew.-% liegt.

Die Aufgabe der Erfindung hat dem entsprechend darin bestanden, ein verbessertes Verfahren zur Herstellung von Extrakten des Ginkgo biloba zur Verfügung zu stellen, welches sich durch höhere Ausbeuten an den drei Wertstoffkomponenten auszeichnet.

### Beschreibung der Erfindung

Gegenstand der Erfindung ist ein Verfahren zur Herstellung von Extrakten des Ginkgo biloba, umfassend die Extraktion der Blätter mit organischen oder wässrig/organischen Lösemitteln, die Adsorption der in den Extrakten enthaltenen Ginkgoflavonoide, Ginkgolide und Bilobalide an Harzen und deren Desorption durch organische Lösemittel, welches sich dadurch auszeichnet, dass man als Adsorbens phenolhaltige Harze einsetzt.

Überraschenderweise wurde gefunden, dass durch Einsatz spezieller Adsorptionsmittel vom Typ phenolhaltiger Harze, speziell vom Typ *"Duolite S-761*" der Firma Rohm & Haas, sich die Ausbeute an Wertstoffen, d.h. an Ginkgoflavonoiden, Ginkgoliden und Bilobaliden signifikant verbessern kann. Da es sich bei den Wirkstoffen um Produkte mit hoher Wertschöpfung handelt, ist jedes Prozent Steigerung der Ausbeute gleichbedeutend mit einem erheblichen Gewinnzuwachs und damit dem entsprechend attraktiv.

### Herstellverfahren

In einer bevorzugten Ausführungsform des erfindungsgemäßen Verfahrens erfolgt die Herstellung der Ginkgo-Extrakte indem man
(a) Blätter des Ginkgo biloba mit wässrig/organischen Lösemitteln extrahiert,
(b) den so erhaltenen ersten Extrakt vom organischen Lösemittel befreit,
(c) das so erhaltene erste Konzentrat zur Abtrennung der Ginkgosäuren einer Flüssig/Fiüssig-Extraktion mit einem aliphatischen Kohlenwasserstoff unterwirft,
(d) den so erhaltenen zweiten Extrakt in einem alkoholischen Lösemittel aufnimmt,
(e) die so erhaltene alkoholische Lösung auf eine Säule aufgibt, die mit einem phenolhaltigen Harz gepackt ist,
(f) das Eluat verwirft und die am Säulenmaterial adsorbierten Flavonoide, Ginkgolide und Bilobalide mit einem wässrig/alkoholischen Lösemittel eluiert, und
(g) das Eluat vom organischen Lösemittelbefreit und trocknet.

Die erste Extraktion der Blätter, die einen Rohextrakt liefert, wird vorzugsweise mit wässrigem Aceton oder Methanol bei Temperaturen im Bereich von 15 bis 40 und vorzugsweise 20 bis 30 °C durchgeführt. Die Einsatzmenge von Blättern und Extraktionsmittel kann 1 : 1 bis 1 : 10 betragen und liegt vorzugsweise im Bereich von 1 : 4 bis 1: 8. Die Extraktionsdauer liegt typisch bei 1 bis 12 und vorzugsweise 4 bis 8 h. Nach Aufkonzentrieren, d.h. teilweisem oder vollständigem Entfernen des Lösemittels und Abtrennen des unlöslichen Rückstands, wird das Konzentrat einer Flüssig/Fiüssig-Extraktion unterworfen, bei der die unerwünschten Ginkgosäuren praktisch vollständig, d.h. bis zu einem Gehalt von maximal 10 ppm entfernt werden. Als Extraktionsmittel kommen vorzugsweise C₅-C₇ Alkane und insbesondere n-Hexan in Frage. Der so erhaltene zweite Extrakt wird in einem alkoholischen Lösemittel, vorzugsweise Methanol oder Ethanol aufgenommen, wobei eine 10 bis 30 Gew.-%ige Lösung hergestellt wird. Die Abtrennung der Wirkstoffe, d.h. der Ginkgoflavonoide, Ginkgolide und Bilobalide erfolgt durch Säulenchromatographie, d.h. die alkoholischen Lösungen werden auf eine Kolonne gegeben, die mit phenolhaltigem Harz als Adsorbens gefüllt ist. Das dabei erhaltene Eluat wird verworfen und die Wirkstoffe anschließend mit wässrigem Alkohol, vorzugsweise wässrigem Methanol eluiert. Das dabei erhaltene Produkt weist einen Aktivsubstanzgehalt von etwa 25 bis 30 Gew.-% auf. Anschließend wird das organische Lösemittel abgezogen und enthaltenes Wasser abgetrocknet. Das resultierende Produkt enthält mindestens 24 und vorzugsweise 25 bis 28 Gew.-% Ginkgoflavonoide jeweils 2,0 bis 5,0, vorzugsweise 2,6 bis 3,2 Gew.-% Ginkgolide und Bilobalide. Ferner weisen die Extrakte weniger als 10 ppm Ginkgosäuren auf.

### Beispiele

**Beispiel 1; Vergleichsbeispiele V1 bis V5.** 1000 g zerkleinerte Ginkgoblätter wurden bei 20 °C in 5 I wässrigem Aceton (50:50) über einen Zeitraum von 6 h dispergiert. Anschließend wurde das erste Extrakt abgetrennt, das Aceton entfernt und das resultierende Konzentrat filtriert. Zur Entfernung von Ginkgosäuren wurde das Filtrat einer Flüssig/Flüssig-Extraktion mit Hexan unterworfen und dem so erhaltenen zweiten Extrakt eine solche Menge Methanol zugesetzt, bis ein Alkoholgehalt von 20 Gew.-% erreicht war. Anschließend wurde die methanolische Lösung auf eine Chromatographiesäule mit unterschiedlichen Packungen gegeben. Die Desorption der adsorbierten Wirkstoffe erfolgte mit wässrigem 90 Gew.-%igem Methanol. Anschließend wurde das Eluat vom Lösungsmittel befreit und getrocknet. In Tabelle 1 sind die Ergebnisse für unterschiedliche Säulenmaterialien zusammengefasst. Angegeben ist jeweils Aktivsubstanzgehalt (Ginkgoflavonoide, Ginkgolide, Bilobalide) nach der Elution (A) sowie im Endprodukt (B)

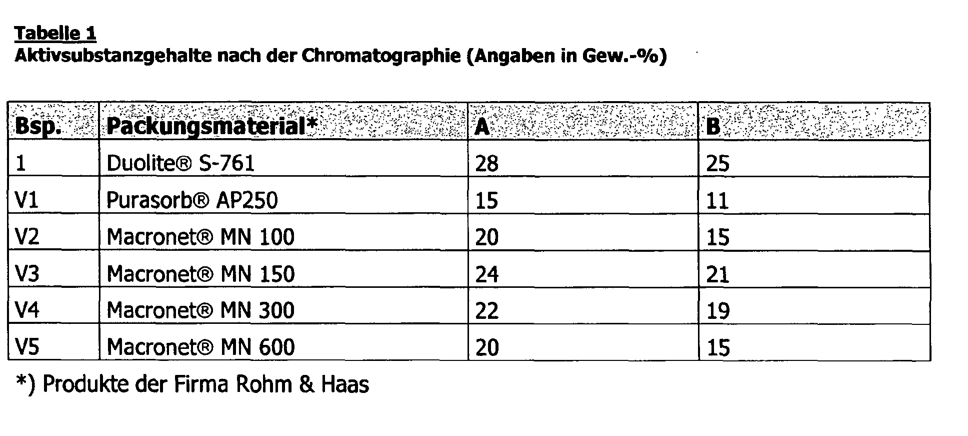

## Patentansprüche

1. Verfahren zur Herstellung von Extrakten des Ginkgo biloba, umfassend die Extraktion der Blätter mit organischen oder wässrig/organischen Lösemitteln, die Adsorption der in den Extrakten enthaltenen Ginkgoflavonoide, Ginkgolide und Bilobalide an Harzen und deren Desorption durch organische Lösemittel, **dadurch gekennzeichnet, dass** man als Adsorbens phenolhaltige Harze einsetzt.

2. Verfahren nach Anspruch 1, **dadurch gekennzeichnet, dass** man phenolhaltige Harze einsetzt, die unter der Marke *Duolite*® *S 761 von* der Firma Rohm & Haas im Handel erhältlich sind.

3. Verfahren nach den Ansprüchen 1 und/oder 2, **dadurch gekennzeichnet, dass** man
(a) Blätter des Ginkgo biloba mit wässrig/organischen Lösemitteln extrahiert,
(b) den so erhaltenen ersten Extrakt vom organischen Lösemittel befreit,
(c) das so erhaltene erste Konzentrat zur Abtrennung der Ginkgosäuren einer Flüssig/Flüssig-Extraktion mit einem aliphatischen Kohlenwasserstoff unterwirft,
(d) den so erhaltenen zweiten Extrakt in einem alkoholischen Lösemittel aufnimmt,
(e) die so erhaltene alkoholische Lösung auf eine Säule aufgibt, die mit einem phenolhaltigen Harz gepackt ist,
(f) das Eluat verwirft und die am Säulenmaterial adsorbierten Flavonoide, Ginkgolide und Bilobalide mit einem wässrig/alkoholischen Lösemittel eluiert, und
(g) das Eluat vom organischen Lösemittelbefreit und trocknet.

4. Verfahren nach Anspruch 3, **dadurch gekennzeichnet, dass** man die Blätter mit wässrigem Aceton oder Methanol extrahiert.

5. Verfahren nach den Ansprüchen 3 und/oder 4, **dadurch gekennzeichnet, dass** man die Fiüssig/Fiüssig-Extraktion mit einem C₅-C₇ Alkan durchführt.

6. Verfahren nach mindestens einem der Ansprüche 3 bis 5, **dadurch gekennzeichnet, dass** man den zweiten Extrakt in Methanol oder Ethanol aufnimmt.

7. Verfahren nach mindestens einem der Ansprüche 3 bis 6, **dadurch gekennzeichnet, dass** man die Wirkstoffe mit wässrigem Methanol eluiert.

8. Verfahren nach mindestens einem der Ansprüche 1 bis 7, **dadurch gekennzeichnet, dass** man Extrakte herstellt, die mindestens 24 Gew.-% Ginkgoflavonoide enthalten.

9. Verfahren nach mindestens einem der Ansprüche 1 bis 8, **dadurch gekennzeichnet, dass** man Extrakte herstellt, die jeweils 2,0 bis 5,0 Gew.-% Ginkgolide und Bilobalide enthalten.

10. Verfahren nach mindesten einem der Ansprüche 1 bis 9, **dadurch gekennzeichnet, dass** man Extrakte herstellt, die weniger als 10 ppm Ginkgosäuren enthalten.
